# EUROPEAN PATENT APPLICATION

(11) **EP 1 470 837 A2**
(43) Date of publication of application: **27.10.2004**
(21) Application number: 04252402.5
(22) Date of filing: 23.04.2004
(51) Int. Cl.: A61N 5/06

(54) **Switched photodynamic therapy apparatus and method**

(30) Priority: 23.04.2003 US 464656 P
(71) Applicant: Tulip, John, Edmonton, Alberta, T6G 1Z7 (CA); MOORE, Ronald B., Edmonton, Alberta T6R 1Z5 (CA); Dickey, Dwayne J., Edmonton, Alberta T6H 2N4 (CA)
(72) Inventor: Tulip, John, Edmonton, Alberta, T6G 1Z7 (CA); MOORE, Ronald B., Edmonton, Alberta T6R 1Z5 (CA); Dickey, Dwayne J., Edmonton, Alberta T6H 2N4 (CA)
(74) Representative: Shanks, Andrew

(57) **Abstract**

A photodynamic therapy apparatus and method in which (1) phototoxic drug is supplied to the arterical supply of a target tissue, (2) deliver of drug activating light to target tissue through probes is controlled by sequential selection of operation of the probes, (3) an automatic radiance probe is used for efficient optical characterization of target tissue and (4) optical dose is monitored by sequential selection of probes as transmitters and receivers.

## Description

### BACKGROUND OF THE INVENTION

In a procedure called Photodynamic Therapy (PDT) a phototoxic drug is combined with light to destroy malignant tumours. Activated phototoxic drugs will react with oxygen, dissolved in tissue, to create a highly reactive form of oxygen called singlet oxygen. Singlet oxygen will oxidize tissue (mainly biomembranes) resulting in cell death and injury. Typically, photosensitive drug is injected intravenously into the patient. After a delay period, needed for the drug to perfuse the tumour and be cleared from normal tissue, the tumour is exposed to light from a lamp or a laser. The wavelength of the light must be suitable to activate the drug. The phototoxic drug Benzo-Porphyrin, for example, has a light activation wavelength of about 680nm.

One limitation to this method is that the optical transparency of human tissues is limited; optical penetration depth is typically only a few millimetres. This depth of penetration is adequate for the treatment of superficial tumours of, for example, the human airway and skin but is too shallow for the treatment of most solid tumours. One method of overcoming this shortcoming is to use fibre optic light sources, known as interstitial light probes or cylindrical probes in the art, implanted into the tumour. In this method the optical probes are typically arranged in a parallel array, each spaced between half and one centimetre from the adjacent probe. In a typical geometry the probes are arrayed in an icosahedral pattern. The cylindrical source probes are typically an integral part of a light delivery fibre optic where the distal end (1-4cm) of the fibre optic is either coated or clad with optical scattering material. This scattering material allows light to radially diffuse from the side of the fibre optic over a length from one up to several centimetres, perpendicular to fibreoptic axis. Light from a probe forms a cylindrical distribution of emission. When a parallel array of cylindrical probes is used to illuminate tissue, variations of light dose occur between the probes. The illumination of tissue is greatest in the vicinity of the source and lowest at a point equidistant from adjacent sources. The illumination along the length of the cylindrical probes depends upon the design of the probe but is relatively uniform.

Light delivery fibre optic cables used for interstitial PDT must be illuminated with a laser since lamp sources cannot be focussed onto the small aperture needed for fibre optic excitation. In the art, light from a single laser is usually split between several fibre optic cables using either beam-splitters or fibre optic splitters. This method of fibre optic illumination is limited because control of illumination of an individual fibre optic is not possible. Biological tissues are unpredictable and significant variations of the optical properties may exist with time and across a tumour. This is the result, for example, of variation in blood profusion and the existence of different structural regions within the tumour. Since exposing all points of a tumour to a lethal light dose is a necessary part of successful therapy and since over exposure of tissue may result in complications associated with damage to normal tissues surrounding the tumour it is highly desirable to control the illumination of an individual fibre optic so as to expose all points of the tissue to lethal light dose.

The problem of achieving a uniformly lethal light dose across a tumour is further complicated by dynamic changes in tissue optical properties over the course of treatment. These changes occur as the result of, for example, damage to blood vessels and consequent changes in blood perfusion. The therapeutic effect of PDT is known to be principally the result of the destruction of blood vessel in and around the tumour. Tumours are well known to have poorly developed vascularization and lymphatic drainage as a result of tumour induced angiogenesis. As a result phototoxic drugs tend to accumulate in the reticuloendothelial system within the tumour. As well, phototoxic drugs tend to accumulate in the walls of blood vessels of all sizes. In addition the oxygen concentration is highest at this site such that photo-toxins associated with PDT cause blood vessel coagulation and collapse. Loss of blood perfusion to the tumour results in ischemia and indirect tumour cell death in addition to direct cell death. The denaturization of tissue over the course of treatment may result in changes of optical properties and a non-uniform application of light dose. Some drugs will act as significant tissue chromophores if they are present in relatively large concentrations. These drugs may also photo bleach and produce a slow increase in tissue transmissivity over the course of treatment.

Complications associated with PDT are associated with damage to vital tissues in the vicinity of the treated tissue. For example, treatment of prostate cancer with PDT carries with it the risk of damage to the rectum. Prostate tumours tend to occur in the posterior part of the prostate, which is adjacent to the rectum. PDT damage to the rectum will be similar to that associated with cryotherapy and can result in fistula and which may create a complex surgical problem for repair. Currently the only method that will prevent collateral damage to surrounding tissues is the control of light dose at the margins of the treatment zone. When injected systemically, known phototoxic drugs distribute approximately uniformly across the patients body tissues, Some evidence of selective accumulation of phototoxic drugs in tumours has been reported but the ratio of drug concentration between the tumour and its surrounding tissues is limited and not of significant therapeutic benefit.

A method of monitoring interstial light dose during PDT has been described in the art. In this device light from a single laser was split, using beam splitters, into six fibre optics connected to interstitial cylindrical probes. A mechanical apparatus was used to obstruct five of the six sources and place an optical detector at the proximal end of the obstructed fibres. Light from the remaining illuminated source was collected by the five obstructed cylindrical probes and the photodetector readings from the five obstructed fibres were used to estimate the uniformity of light dose throughout the tissue. This apparatus has the limitation that the light dose to each cylindrical source may not be controlled. Moreover the photodetector switching apparatus is relatively complicated and slow and requires direct current motors, gearboxes and friction clutches to swing the gate like structures in place. This apparatus provided an indication of the uniformity of light dose but provided no means of correcting an inhomogeneous dose distribution. Because of variations of light dose among the probes, because of beam splitting variations and because of detector variations it was necessary to calibrate this system using a bath of intratipid. This procedure is not compatible with clinical practice. A short treatment period is required for inter-arterial drug delivery and some modem PDT drugs, which are active for only minutes following administration, so in these cases a fast dose monitoring protocol is essential.

### SUMMARY OF THE INVENTION

We disclose here several methods and apparatus for the treatment cancer that overcomes limitations of conventional PDT. According to an aspect of this invention, phototoxic drug is not applied intravenously but is applied to the arterial system of the target, followed by illumination of the target tissue by drug activating light. There is also provided in accordance with an aspect of the invention, an apparatus for performing photodynamic therapy of a target tissue having an arterial supply, the apparatus comprising a source of phototoxic drug, a drug injector having a needle for injection of the phototoxic drug into the arterial supply, the drug injector being connected to the source of phototoxic drugt; and a photo-dynamic light source arranged to provide drug activating light to the target tissue. The phototoxic drug preferably has a first-pass effect.

We further disclose a method of achieving a uniformly lethal light dose to the target tissue, while monitoring in real time light and drug dose. There is therefore providing in accordance with an aspect of the invention, an apparatus for delivering drug activating light to target tissue, the apparatus comprising plural probes; and a drug activating light delivery system arranged to cause the plural probes, in operation, to sequentially deliver drug activating light to the target tissue. In addition there is a detection device for drug levels and light dose. The drug activating light delivery system may comprises a laser having drug activating light emission, an optical switch optically coupled between the laser and the plural probes, the optical switch having plural operating positions corresponding to connection of the laser to respective ones of the plural probes; and a controller for operation of the laser and the optical switch. Several lasers may be coupled to the probes. There is also provided a method for delivering drug activating light to target tissue, the method comprising the steps of placing plural probes in sufficient proximity to the target tissue to direct drug activating light towards the target tissue and activate drug in the target tissue; and providing drug activating light from at least one laser to the plural probes sequentially.

Still further, we disclose an apparatus, called an automatic radiance probe, which may be used to perform radiance measurements very rapidly and communicate these measurements to a control computer. Therefore, according to an aspect of the invention, there is provided an apparatus for delivering light to target tissue, the apparatus comprising a light delivery fiber terminating in a radiance probe, a chuck for securing the light deliver fiber, a motor for rotating the chuck; and a motor control operably connected to the motor. If optical properties are determined throughout the tissue using this apparatus, the light dose needed to achieve a homogeneous light dose throughout the tissue, may be predicted. This predicted dose distribution allows treatment planning prior to therapy.

Still further, we describe an apparatus and method for mapping the optical characteristics of a solid tissue body in a time period that is clinically practical. The apparatus comprises the radiance probe in combination with an array of probes, and a computer for receiving and analyzing light intensity signals obtained from the probes. In the method of characterizing optical properties of a target tissue for photo-dynamic therapy, there are carried out the steps of placing an array of probes, for example in a human body, placing a directional probe in the human body with target tissue between the directional probe and the array of probes, rotating the directional probe, detecting intensity of light that has passed between the directional probe and respective probes in the array of probes; and computing optical properties of the target tissue from the detected light intensity. In a further aspect of the invention, there is provided the step of, after computing optical properties of the target tissue at a first axial location, advancing the directional probe in the axial direction and computing optical properties of the target tissue at a second axial location. The probes are preferably located in therapeutic position within suitable needles. Each probe in the array is preferably illuminated sequentially as the directional probe rotates by operation of a stepper motor.

In a still further aspect of the invention, a method and apparatus are disclosed for monitoring radiation dose applied during photodynamic therapy. In this aspect of the invention, light from one set of probes is received by another set of probes located with target tissue between the sets of probes. As probes of one set of probes transmit, the dose applied by those probes is detected by the other set of probes. Which probes act as transmitters and which probes act as receivers is switched to measure the dose applied by both sets of probes.

Further summary of the invention may be found in the detailed disclosure that follows and the claims.

### BRIEF DESCRIPTION OF THE DRAWINGS

There will now be described preferred embodiments of the invention with reference to the figures, for purposes of illustrating examples of the invention, in which:
Fig. 1 shows an apparatus for delivery of drug activating light to plural probes, including a detector for use in dose monitoring;
Fig. 2 shows application of phototoxic drug to the arterial supply of a tumour;
Figs. 3A and 3B shows an automatic radiance probe for use with an embodiment of the invention;
Fig. 3C shows a prior art radiance probe;
Fig. 4 is a flow chart of a method of treatment;
Fig. 5 is a flow chart of another method of treatment; and
Fig, 6 is a flow chart of a method of characterizing the optical properties of tissue.

### DETAILED DESCRIPTION OF PREFERRED EMBODIMENTS

In this patent document, the word comprising is used in its inclusive sense and does not exclude other elements being present. The indefinite article "a" before an element also does not exclude more than one of the element being present. The term "light" or "drug activating light" refers to electromagnetic radiation of a wavelength suitable for drug activation, for example phototoxic drug activation. An "optical" element is an element capable of transmitting and guiding drug activating light. The term "probe" refers to a device capable of delivering drug activating light to target tissue. Probes are typically connected to laser light sources through optical fibres. A probe may also be used as a receiver of light when the probe is connected to a detector. A "phototoxic drug" is a drug that is activated by application of light, and includes lypo-phyllic drugs. The phototoxic drug preferably has a first pass effect, in which most of the drug is taken up in the targetted tissue on it's first pass through.

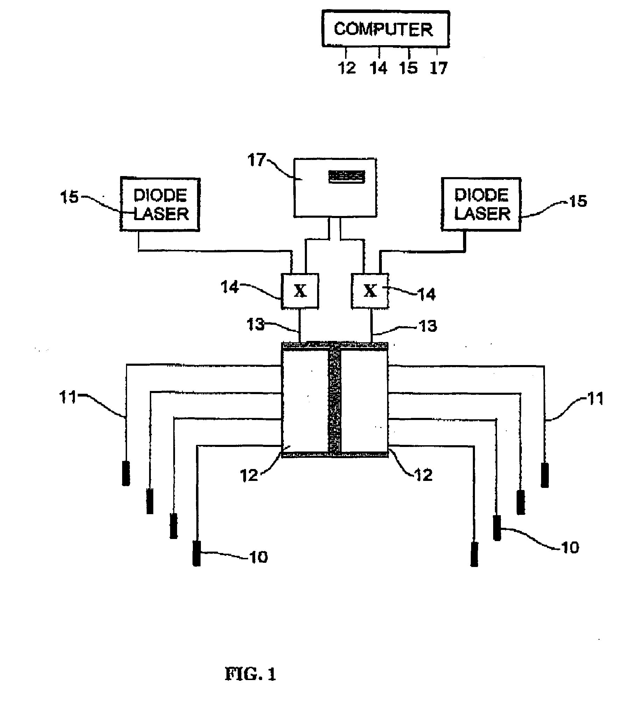

Apparatus for achieving a uniformly lethal light dose to target tissue is shown in Fig. 1. An array of probes 10 are coupled to a drug activating light delivery system. In this example, the drug activating light delivery system comprises optical fibers 11 leading from the probes 10 to optical switch 12, which in turn is coupled through optical fibers 13 to detector switch 14 and from there to laser 15, or other suitable drug activating light source, A computer 16, with suitable labelled output ports 12, 14, 15 indicating to which element the ports are connected, is used to control operation of the switches and laser. The detector switch 14 is not required for uniform dose application, but is used for dose monitoring, as described below. The optical switch 12 is for example an 1xm fiberoptic switch, where m is the number of probes 10, for example 4. An identical set of probes 10 and drug activating light delivery system composed of elements 11, 12, 13 and 14 are also shown in Fig. 1 and used for dose monitoring as described below.

The probes 10 are preferably cylindrical probes inserted interstitially through conventional associated needles into a tumour or diseased. organ as is currently practiced in the art. Unlike conventional practice, however, light from one or more lasers 15 is not split between the cylindrical probes 10 in the array but is switched sequentially between the probes 10. In a preferred operation of this method, all of the light from a laser 15 is coupled to a single probe 10 until the laser 15 is switched to another probe 10. The fibreoptic switch 12 and computer 16 controls the sequence, exposure time and laser power for each probe 10 in the array.

This method provides several benefits over the currently practiced art. Since the exposure time and illumination of each probe 10 may be varied, the dose of light delivered to a probe 10 and its surrounding tissue may also be varied. The light dose may be increased in relatively opaque parts of the tissue body and decreased in relatively transparent parts in order to achieve a uniform light dose. This method may consequently concentrate light dose in refractory parts of the tissue without over exposing the rest of the tumour and risking collateral damage to surrounding tissues.

This method of fibreoptic switching, moreover, has the added advantage of potentiating the therapeutic effect of PDT. It is well known in the art that PDT is associated with the depletion of tissue oxygen since the therapy is essentially one of photo-oxidation of tissue. Although depletion of some phototoxic drugs may occur as a result of photochemical dissociation, the role of the drug is principally to catalyze photo-oxidation of tissue. Reoxygenation of tissue occurs through the perfusion of blood. In the method of switched light delivery, tissue may be exposed for a first period until de-oxygenation causes saturation of therapy. Following this, a second period of no light exposure may allow re-oxygenation before the therapy is continued. This cycle is repeated until the required total dose is delivered to all probes 10 in the array. This cycle of oxygen depletion and repletion is of course limited in those tissue where illumination is mainly from one probe 10. For regions in the tissue midway between several probes the tissue will have less time to reoxygenate between light exposures.

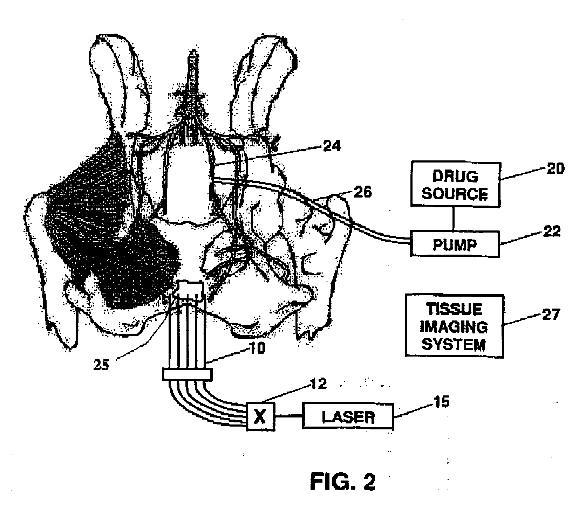

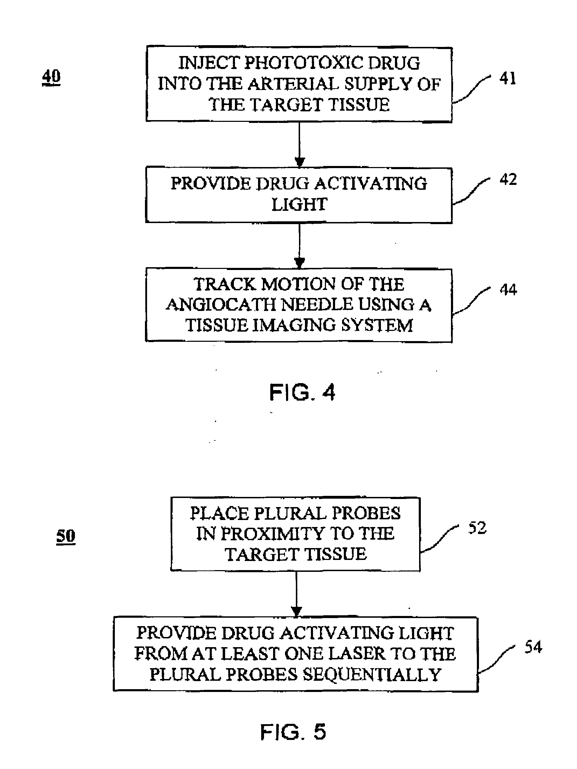

In a preferred example of operation of apparatus used for PDT, drug is not applied intravenously, but is applied to the arterial system of the target tissues using angiographic radiological techniques. For example, as shown in Fig. 2, a source 20 of phototoxic drug is connected to supply drug to a drug injector 22 for injection of the phototoxic drug into the arterial supply 24 of a tumour 25. A photo-dynamic light source, here shown by laser 15, switch 12 and probes 10, is arranged to provide drug activating light to the target tissue. The drug injector may for example be a pump 22 that pumps drug into the arterial supply 24 using a drug delivery tube 26 that terminates in an angiocath needle (not shown) inserted through a tracker small vessel catheter (not shown). Preferably, a tissue imaging system 27, such as a conventional electromagnetic (radiographic) imaging system located near the patient, is used for coordinating motion of the angiocath needle in conventional fashion. The phototoxic drug may for example be Benzo-Porphyrin or Hypocrellin or other lypo-phyllic drug, and the target tissue may be the prostate. Referring now to Fig. 4, the method of use of the above example is described, denoted generally by reference character 40. The phototoxic drug is injected into the arterial supply of the target tissue in step 41, and is activated by light in step 42. The motion of the angiocath needle at the end of the drug delivery tube 26 is tracked using the tissue imaging system in step 43.

Lypo-phyllic drugs will aggregate in the blood stream, temporarily embolize and adhere effectively to the small capillaries in the vicinity of the intra-arterial injection point. Application of drug to the arterial supply of the target tissue may consequently be used to achieve high concentration of the drug in the target tissue and low concentration in surrounding tissues that draw blood supply from other parts of the vascular system. For example, application of drug to the arterial supply of prostate will result in the concentration of phototoxic drug within prostate, which is over one hundred times greater than that in surrounding tissues such as the rectum and urethra. This technique should therefore protect vital surrounding tissues over the course of PDT and significantly reduce the incidence and risk of complication. This protection should persist until the drug leaks from the target tissue and is distributed systemically to surrounding organs and tissues. The period of selective drug uptake is adequately long for PDT treatment following intra-uterial application of drug.

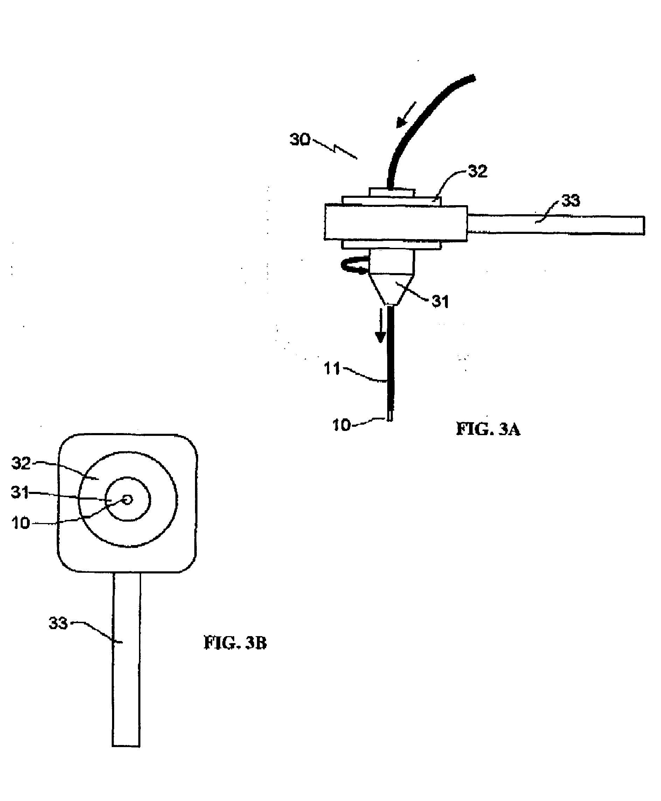

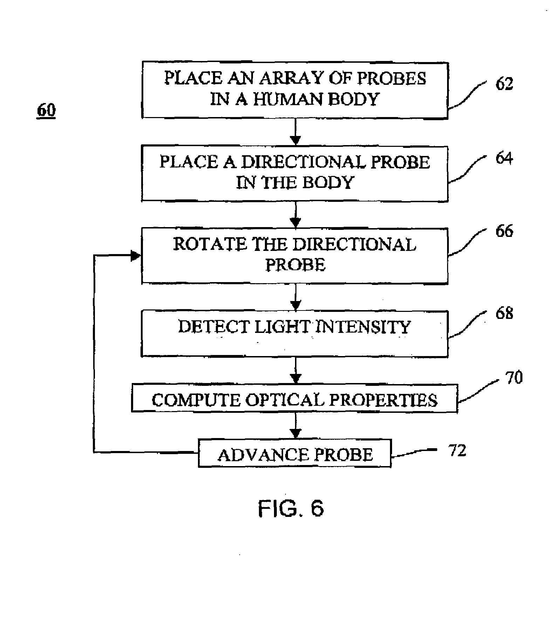

PDT may be made more effective through treatment planning. Switched fibre optic light delivery by apparatus illustrated in Fig. 1 may be used to control the light dose needed to overcome the optical inhomogeneity of malignant tissue. In order to plan a treatment prior to therapy, the distribution of optical properties throughout the tissue body must be known. Optical properties may be determined using a method known in the art as the P3 Approximation in conjunction with the measurement of tissue radiance. An automatic directional radiance probe 30, with fiber optic 11 terminating in radiance probe 10, chuck 31, motor 32 and handle 33, shown in Figs. 3A and 3B, may be used to perform radiance measurements very rapidly and communicate these measurements to a control computer. If optical properties are determined throughout the tissue using this apparatus, the light dose needed to achieve a homogeneous light dose throughout the tissue, may be predicted. This predicted dose distribution allows treatment planning prior to therapy. Referring now to Fig. 6, a method of characterizing the optical properties 60 is described. In step 61, an array of probes is placed in a human body. In step 62, a directional probe is placed in the human body with target tissue between the directional probe and the array of probes. The directional probe is rotated in step 63, and in step 64, light intensity of light that has passed between the directional probe and respective probes in the array of probes is detected. The optical properties of the target tissue are then computed from the detected light intensity in step 66. The probe is then advanced in step 66, and the optical properties of the new location are also computed by returning to step 63.

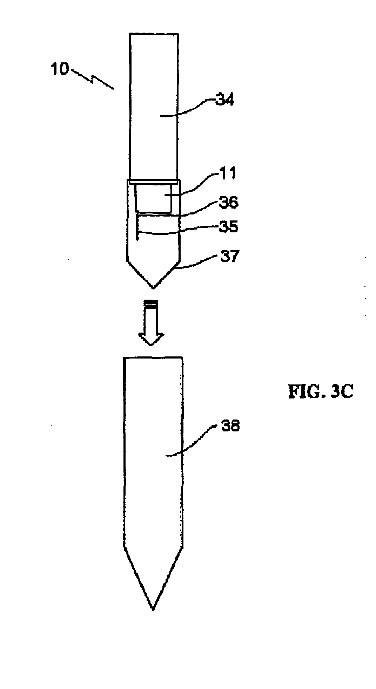

As described in the art, a conventional radiance probe may be used to characterize the optical properties between the probe and a small spherical light source. Fig. 3C shows a sketch of a radiance probe 10, which includes a fiber optic 11, with conventional coating 34, for delivering light to and receiving light from the radiance probe 10. At the end of the radiance probe 10 a coated right angle prism 35 is attached with optical epoxy 36 and protected with a protective glass dome 37. The probe 10 is inserted into an afterloading needle 38. The radiance probe 10 will detect a maximum of scattered light when orientated in the direction of the light source and a minimum of scattered light when orientated 180 degrees away from the source. The distribution of detected light between these two extremes is called a radiance characteristic and this may be combined with the P3 Approximation to determine tissue optical properties between the source and the probe. Radiance probes described, in the art are of very little clinical utility. Prior art radiance probes must be oriented toward the measurement path. The probe and the source must have the same axial position and the orientation of the probe with respect to the path must be known. In order to map the optical characteristics of a solid tissue body many paths must be characterized and prior art radiance probes are too slow and laborious to be clinically practical.

A novel apparatus and method for mapping the optical characteristics of a solid tissue body in a time period that is clinically practical is now described. Conventional transparent needles are implanted into the tissue body under acoustic or fluoroscopic imaging guidance. These needles are placed in a parallel array such that when cylindrical probes 10 are introduced into the needles, the cylindrical probes 10 will be of suitable length and suitable spacing for effective therapy. Prior to treatment the automatic radiance probe 30 is used to map the optical properties of the target tissue.

The radiance probe 30 is motorized with stepping motor 32 under the control of computer 16 or another computer. The fibreoptic cable 11, attached to the radiance probe 30, is inserted and clamped using chuck 31, in the rotating head of the stepper motor 32, as illustrated in Fig. 3A. The chuck 31 rotates under control of the motor 32 between positions 180 degrees each side of a central position. This allows full 360 degree coverage without risking breakage of the optical fiber, which needs to twist as the chuck 31 is rotated. The stepper motor 32 and radiance probe 30 are attached to a handle 33 so that an operator may manually insert the radiance probe 10 into the conventional transparent needle (not shown). The handle 33 of the probe 30 also contains a small microprocessor needed to coordinate the rotation of the probe 30 with the rest of the apparatus. Date may be acquired for example at every 10 degrees over each 180 degree sweep. Cylindrical probes are placed in the transparent needles adjacent to the needle containing the rotating radiance probe 30. Referring now to Fig. 5, the method of use 50 is shown. The radiance probes 50 are placed in proximity to the target tissue in step 51. In step 52, the radiance probe 30 and the adjacent cylindrical probes 10 are synchronized so that typically four adjacent. probes are sequentially illuminated as the probe 30 rotates four times and records the radiance data for the four paths between the probes 10 and the radiance probe 30. The radiance probe 30 is advanced axially by movement of the probe 30 through the needle into which it is inserted, and the radiance (light intensity) measurement is repeated for typically three to four points along the length of the transparent needle. The probes 10 or radiance probe 30 may be used as either transmitter or receiver.

This method, which makes use of a cylindrical light source 10 rather than a spherical source consequently avoids the time consuming step of aligning a source and the radiance probe 30 at a specific axial position. The computer records the measured radiance characteristics and those characteristics are aligned in software with a stored normalized radiance characteristic. This avoids the step of mechanical angular orientation needed with conventional radiance probes. The computer compares radiance characteristics with the P3 Approximation and the optical characteristics of the tissue between the cylindrical source and the radiance probe are computed and stored. The radiance probe 30 may be introduced into each needle and the resulting data may be used to map optical parameters in three dimensions. Tissues found to be more inhomogeneous require more measurement points than those relatively free from inhomogeneity. The disclosed method and apparatus consequently avoids the orientation and positioning steps required by prior art radiance probes and automatically computes optical characteristics a solid tissue body. The time required for optical mapping of for example the human prostate depends upon the number of sources used and the homogeneity of the tissue but is typically in the order of minutes.

Following the characterization of the tissue body, described above, a treatment plan is computed. The computer 16 uses an optical transport model and the measured tissue parameters to predict the light fluence that will result from illumination of the tissue body by the array of cylindrical probes 10. The distribution of light- dose delivered by the cylindrical probe array needed to produce a uniform and lethal light- dose at all points is then calculated. The dose is controlled by either time or light level fractionation. In the method of time- fractionation the laser power is held constant and the time period, for which each cylindrical source is connected to the laser, is varied. The longer the connected time, the greater will be the integrated light-dose to tissue surrounding the cylindrical probe 10. In the method of light level fractionation the connection time to each cylindrical probe 10 is held fixed and the laser power delivered to the cylindrical probes 10 is varied. Although time fractionation is technically easier than light level fractionation the exposure period for each source must be chosen so that reoxygenation of tissue occurs between sequential treatments and this requirement limits the time fractionation protocol.

Although treatment planning is an essential part of any physical treatment modality, this planning process has limitations, Cylindrical probes 10 do not provide control of light emission along the length of the probe and typical cylindrical probes have significant variation in emission along their length. A planning protocol that factors in manufacturing variation of cylindrical probes is too slow and complex. For fast acting drugs characterization must be performed prior to treatment. Phototoxic drugs when present in tissue become chromophores and, in high enough concentration, will change the optical properties of the tissue. Drug bleaching and PDT induced tissue changes are known to change tissue light transmissivity over the course of treatment. It is consequently highly desirable to have a method of real time tissue light dose monitoring so that the evolution of light dose may be monitored and modified if necessary from the beginning to the termination of treatment.

A novel dose monitoring apparatus that overcomes limitations of prior art devices will now be described. The method uses fibreoptic switches 12 and 14 shown in Fig. 1 to integrate the functions of dose delivery and dose monitoring. The switches 12 and 14 may for example be bidirection fiberoptic switches available from LIGHTech Fiberopties Inc. connectorized with SMA905 connectors. The apparatus may be used to interactively adjust light dose delivery to tissue to ensure a uniformly lethal light dose. Fibre optic light switches 12 and 14 are commonly used in the telecommunications art. They are very fast, reliable, have reproducible performance and are mass-produced. Manufacturers of fibre optic switching apparatus routinely mount customized switches and fibre optic lasers within a single rack mounted enclosure and provide a single digital input for remote computer control, such as by computer 16. Such a fibre switch enclosures are compatible with clinical practice. The external connections to such an enclosure are an array of fibre optics 11, 13 terminated with cylindrical probes 10 and a single digital control line,

Fig. 1 shows a typical embodiment of this apparatus. The external fibreoptics 11, 13 are connected to two 1xN switches 12. In the example shown, N=4, The single ends of these switches are connected to two 1x2 switches 14. One side of the 1x2 switches 14 connects to a fibreoptically-coupled laser 15 and the other side of the 1x2 switch 15 connects to a fibreoptically-coupled photodetector 17, for example a detector with high sensitivity such as a 152 mm integrating sphere detector available from Melles Groot. In order to coordinate the physical distribution of illumination with the computer controls, each fibre 11, 13 is given a physical number and computer number. Fibreoptics 11 from the two 1x4 switches 14 are interleaved so that they are connected to adjacent cylindrical probes 10.

In therapeutic mode both lasers 15 are connected to the cylindrical probes 10. Both lasers 15 switch sequentially between four probes 10 so that two probes 10 are simultaneously illuminated. The delivery sequence of the two lasers 15 is chosen to minimizes the volume of tissue illuminated by both lasers 15. The computer 16 controls the light dose delivered to each point in the predetermined manner described earlier. Both the power output of the two lasers 15 and the exposure time of each probe 10 may be varied to achieve the desired light dose distribution.

In tissue monitoring mode one of the 1x2 switches 14 is connected to the detector 17 (a photodiode for example) and the other is connected to a laser 15. The cylindrical probes 10 connected to the photodiode 17 collect light when the probes 10 connected to the laser 15 are sequentially illuminated. The collected light is transmitted along the fibre optic 18, monitored by the photo detector 17 and recorded by the computer 16. In typical animal tissues, light fluence falls exponentially with a penetration depth of a few millimetres so only those cylindrical probes adjacent to the illuminated probe collect a significant light level. Following this the 1x2 switches are thrown and the sequence is reversed between laser 15 and detector probes 10. The computer 16 uses these measurements to estimate the optical extinction coefficient between each of the cylindrical probes 10. This information is combined with a light diffusion model and the light dose applied to each probe 10 during therapy to create a two-dimensional plot of light dose over a plane normal to the probes. This of course is an imaginary plane that represents the average dose along the length of the probes 10. The computer 16 may then, if necessary, iterate the probe dose to correct for regions of low light dose and a new delivery protocol, which differs from the planned protocol, may be implemented at the operators discretion. Because fibre optic switches may be switched in milliseconds the time needed to perform dose monitoring and information is only seconds.

An example of a treatment cycle would have one 2x1 switch switched to one laser source and the desired output fiber of the related 1x9 switch would be chosen to deliver light to the tissue. Meanwhile, the other 2x1 switch would be selected to detection and the nearby fibers to the source would be scanned and an optical power measurement would be taken at each location. The power at each location is then communicated and recorded by the computer, thus allowing for real-time dose monitoring. This continues until each fibre in the array has been used as a source and the corresponding light levels in nearby tissue measured. Note that the switching speed for the optical switch is on the order of 150ms, which is negligible when compared to the total treatment time. The cycle repeats until the desired dose level has been reached.

The lasers used may operate at for example 690 nm or 532 nm, and may be obtained for example from Optical Fiber Systems Inc., including laser diode, driver electronics and cooler. The laser wavelength is largely dictated by the chosen photosensitizer. OS-QLT-0074 may be used, which shows strong absorption at 690 nm. This wavelength penetrates more through the prostate than light at 630 nm. The fiber optics may be terminated with 15 mm cylindrical diffusing tips available from Polymicro Technologies. An icosahedral pattern of the probes may be used to assist with dose uniformity, in which pattern the probes are equally space around target tissue approximately at the corners of an icosahedron.

Immaterial modifications may be made to the embodiments described here without departing from the invention.

## Claims

1. An apparatus for performing photodynamic therapy of a target tissue having an arterial supply, the apparatus comprising:
a source of phototoxic drug;
a drug inject for injection of the phototoxic drug into the arterial supply, the drug injector being connected to the source of phototoxic drug; and
a photo-dynamic light source arranged to provide drug activating light to the target tissue.

2. The apparatus of claim 1 in which the photo-dynamic light source comprises plural probes and a drug-activating light delivery system arranged to cause the plural probes, in operation, to sequentially deliver drug activating light to the target tissue.

3. An apparatus for delivering, drug activating light to target tissue, the apparatus comprising:
plural probes; and
a drug-activating light delivery system arranged to cause the plural probes, in operation, to sequentially deliver drug-activating light to the target tissue.

4. The apparatus of claim 2 or 3 in which the drug-activating light delivery system comprises:
a laser having drug-activating light emission;
an optical switch optically coupled between the laser and the plural probes, the optical switch having plural operating positions corresponding to connection of the laser to respective ones of the plural probes; and
a controller for operation of the laser and the optical switch.

5. The apparatus of claim 4 further comprising at least one additional laser, each having drug-activating light emission, the optical switch being optically coupled between each of the lasers and the plural probes.

6. The apparatus of claim 4 or 5 in which the controller comprises laser output power control.

7. The apparatus of claim 4, 5 or 6 in which the controller is configured to control the optical switch to deliver drug activating light to the probes sequentially.

8. The apparatus of claim 4, 5, 6 or 7 further comprising:
a drug-activating light detector;
a detector switch in the light path between the laser and the optical switch, the detector switch and optical switch having operating positions in which the laser is optically coupled to at least one probe to act as a transmitter and at the same time the drug-activating light detector is optically coupled to at least a different one of the probes to act as a receiver; and
the controller is operably connected to the detector switch and the detector to control selection of the operating positions and record detected light for characterization of optical characteristics of the target tissue.

9. The apparatus of claim 8 further comprising at least a second laser and at least a second detector switch optically coupled between the second laser and the optical switch, the second detector switch and optical switch having operating positions in which the second laser is optically coupled to at least one probe to act as a transmitter.

10. The apparatus of any one of claims 1-9 further comprising a tissue imaging system for coordinating motion of the drug injector.

11. The apparatus of any one of claims 1-10 in which the phototoxic drug is Benzo-Porphyrin or Hypocrellin.

12. The apparatus of any one of claims 2-11 in which the target tissue is the prostate gland of a human being, and the drug activating light is applied to the target tissue through probes.

13. An apparatus for delivering light to target tissue, the aparatus comprising:
a light delivery fiber terminating in a radiance probe;
a chuck for securing the light deliver fiber;
a motor for rotating the chuck; and
a motor control operably connected to the motor.

14. The apparatus of claim 13 used in connection with an optical switch, the control being configured to synchronize the optical switch with rotation of the chuck.

15. An optical dose monitoring apparatus, comprising:
two or more lasers, each laser having drug-activating light emission;
each laser being optically coupled to a detector switch;
the detector switch being optically coupled to an optical switch and a photo-detector;
the optical switch being coupled to plural probes;
the detector switch and optical switch having operating positions in which selected ones of the probes act as transmitters of drug activating light from the lasers while different selected ones of the probes act as receivers of light scattered from target tissue, the scattered light being supplied to the photo-detector; and
a controller for controlling the operating positions of the detector switch and operating switch.
